# EUROPÄISCHE PATENTSCHRIFT

(11) **EP 1 890 620 B1**
(45) Veröffentlichungstag und Bekanntmachung des Hinweises auf die Patenterteilung: **09.01.2013**
(21) Anmeldenummer: 06721952.7
(22) Anmeldetag: 09.05.2006
(51) Int. Cl.: A61B 17/68, A61B 17/72, A61B 17/00

(54) **VORRICHTUNG FÜR DIE OSTEOSYNTHESE**
OSTEOSYNTHESIS DEVICE
DISPOSITIF D'OSTEOSYNTHESE

(30) Priorität: 13.05.2005 CH 8482005
(43) Veröffentlichungstag der Anmeldung: 27.02.2008
(73) Patentinhaber: Klaue, Kaj, 6942 Savosa (CH)
(72) Erfinder: Klaue, Kaj, 6942 Savosa (CH)
(74) Vertreter: Lusuardi, Werther
(86) Internationale Anmeldenummer: PCT/CH2006/000250
(87) Internationale Veröffentlichungsnummer: WO 2006/119659

(56) Entgegenhaltungen:
- WO-A-2004/089255
- DE-A1- 3 919 900
- US-A- 1 951 278
- US-A- 4 805 607
- US-A- 4 898 186
- US-B1- 6 551 343

## Beschreibung

Die Erfindung bezieht sich auf eine Vorrichtung gemäss dem Oberbegriff des Patentanspruchs 1, und auf einen eine Ausführungsform der Vorrichtung und einen Bohrer umfassenden Kit gemäss Anspruch 16.

Solche Vorrichtungen können für verschiedene medizinische Indikationen verwendet werden, insbesondere für:
A) die Fixation von Gelenksfragmenten, d.h. von Fragmenten, welche sowohl Knochen- als auch Knorpelanteile aufweisen; sowie
B) zur temporären Schienung von Zehen, insbesondere zur Behandlung von Hammerzehen oder anderen Fehlstellungen von Zehen.

Gelenksfragmente treten beispielsweise in folgenden Fällen auf:
a1) bei Unfällen, z.B. kombiniert mit Bänderverletzungen und eventuellen Verrenkungen;
a2) bei chronischen Gelenkinstabilitäten;
a3) bei Wachstumsstörungen beim Adoleszenten (sogenannte Osteochondrosis oder Osteochondritis).

Betroffen sind in diesen Fällen meist das Knie (Femur), das obere Sprunggelenk (Talus) und das Hüftgelenk (Femur).

Gelenksfragmente messen typischerweise zwischen 2 und 30 mm und müssen zur einwandfreien Funktion des Gelenks anatomisch genau fixiert werden. Dabei ist es wesentlich, dass das Gelenk nicht ruhig gestellt wird wegen der Knorpelernährung. Es empfiehlt sich eine postoperative Behandlung mit kontinuierlicher, passiver Bewegungstherapie (CPM = continuous passive motion). Im weiteren muss das Gelenksfragment bewegungsfrei mit seinem Grundknochen stabil verbunden bleiben. Operationen für die oben unter A) genannten Indikationen nennen wir "Osteochondrosynthesen". Dabei sind besondere sogenannte intrafragmentäre Scher-Bewegungen gefürchtet. Um diese zu verhindern werden solche Fixationen mit transfragmentären Stiften durchgeführt, welche von der Gelenksseite in den epiphysären Knochen eingeführt werden. Es ist auch bekannt solche Stifte aus resorbierbarem Material zu fertigen.

Die Gelenksfragmente sind zumeist so klein, dass oft nur ein einziger Stift darin plaziert werden kann. Mehrere Stifte würden auch die Festigkeit und die Durchblutung des Knochenanteils gefährden. Im weiteren sind die Gelenksfragmente oft so gelegen, dass sie nur schwierig von orthogonal und vom Gelenk her angegangen werden können.

Stand der Technik für die oben unter B) genannten Indikationen ist die Kirschnerdraht-Fixation der Zehengelenke während der Heilungszeit (Weichteil- und/oder Knochenheilung) wobei der Draht an der Zehenspitze hervorragt. Der Nachteil dieser bekannten Technik besteht darin, dass der Patient kaum arbeitsfähig ist, da er eine gewisse "Stosstange" (z.B. eine harte Schiene) tragen muss.

Die häufigste Operation dieser Art ist die Arthrodese des proximalen Interphalangeal-Gelenkes, d.h. das Zusammenwachsen der Knochen, wobei leider oft nur die Gelenkresektion durchgeführt wird (sogenannte Operation nach Hohmann). Es wird auch die funktionelle Operation empfohlen, wobei Sehnen der Endphalanx auf die Grundphalanx transferiert werden (sogenannte Operation nach Girdlestone und Taylor 1947). Beide Operationen benötigen 6 bis 8 Wochen mechanische Ruhigstellung.

Aus der W02004/089255 ist eine rohrförmige Vorrichtung zur temporären Schienung von Zehen bekannt, welche mittels eines Führungsdrahtes implantiert wird. Diese bekannte Vorrichtung besitzt jedoch verschiedene Nachteile, nämlich:
- der runde Querschnitt des Rohres führt dazu, dass die einzelnen Knochen sich um das Rohr drehen können, d.h. es fehlt eine Rotationssicherung des Implantates;
- eine aufwendige Operationstechnik (der eingeführte Führungsdraht kann sich verbiegen und einen falschen Weg passieren; das Rohr kann sich auf dem Führungsdraht verklemmen; das rohrförmige Implantat und der Führungsdraht sind in sich selbst geschwächt (kleine Dimension des Drahtes und zentrale Kannulierung im Rohr; die Anwendung von distal her, d.h. von der Zehenbeere ausgehend, opfert das distale Interphalangeal-Gelenk).

Für die oben geschilderten Probleme will die Erfindung Abhilfe schaffen. Der Erfindung liegt die Aufgabe zugrunde, eine Vorrichtung zu schaffen, mit der es möglich ist folgende Ziele zu erreichen:

Für die unter A) genannten Indikationen:
A1) eine rotations- und gleitfeste Fixierung des Gelenksfragmentes Mittels eines einzigen Stiftes;
A2) die Verwendung eines adäquaten Bohrmaterials (alternierend rotierender , flexibler Bohrer) und das Anbohren durch eine gebogene Bohrhülse;

Für die unter B) genannten Indikationen
B1) eine Rotationsstabilität des Implantats zu garantieren, um damit die Arthrodese in einer richtigen, stabilen Stellung konsolidieren zu können und somit eine natürliche Position des Zehennagels und der Zehenbeere zu erwirken;
B2) eine Hyperflexions-Fehlstellung des proximalen Interphalangeal-Gelenks zu korrigieren;
B3) den Bodenkontakt der Zehenbeere zu gewährleisten;

Die Erfindung löst die gestellte Aufgabe mit einer Vorrichtung, welche die Merkmale des Anspruchs 1 aufweist, sowie mit einem eine Ausführungsform der Vorrichtung und einen Bohrer umfassenden Kit, welcher die Merkmale des Anspruchs 16 aufweist.

Die durch die Erfindung erreichten Vorteile sind im wesentlichen darin zu sehen, dass dank der erfindungsgemässen Vorrichtung:
a) das nicht involvierte Gelenk (distales Interphalangeal-Gelenk) ausgespart werden kann;
b) die interfragmentäre Stabilität drastisch verbessert wird und damit die Konsolidation gesichert wird;
c) die Stabilität insbesondere in ROTATION gehalten wird.

Bei einer besonderen Ausführungsform ist der unrunde Querschnitt des Stabes nur auf einem Teil seiner Gesamtlänge realisiert. Damit kann die Festigkeit optimiert werden. Der unrunde Querschnitt kann polygonal, vorzugsweise dreieckig ausgebildet sein. Durch die Form dieses Profils ergibt sich eine Optimierung der Rotationssicherung.

Bei einer weiteren Ausführungsform der Erfindung kann die Zentralachse des Stabes gekrümmt sein. Der Vorteil dieser Ausgestaltung liegt im wesentlichen darin, dass die anatomische Achse der Zehe in der sagittalen Achse rekonstruiert wird.

Der Bodenkontakt der Zehenbeere wird mit einer leichten "Vorspannung" in Beugung gebracht. Als vorteilhaft hat sich erwiesen die Tangenten an den beiden Endpunkten der Zentralachse des Stabes unter einem Winkel von 5° - 20° schneiden zu lassen. Der Vorteil dieser Ausführung liegt in einer "Nagelung" des Gelenksfragmentes oder des Röhrenknochens, welche bei schweren Zugängen von einer beliebigen Seite her durch eine Einschlagshülse erfolgt.

Der erfindungsgemäße Stab weist auf seiner Mantelfläche mindestens drei Längskanten oder Längsgräte auf. Damit ist der Vorteil erreichbar, dass sich die Profilkanten des Stabes im Knorpel- und im Knochengewebe festschneiden.

Die Längskanten oder Längsgräte sind durch konkave Vertiefungen voneinander getrennt.

Der maximale Aussendurchmesser des Stabes beträgt vorteilhafterweise 1,5 - 3,5 mm, der Kerndurchmesser 1,0 - 2,5 mm.

Bei einer weiteren Ausführungsform der Erfindung verjüngt sich der Stab in Richtung des Zentrums des Krümmungsradius der Zentralachse. Dadurch kann der Stab leichter implantiert werden.

Der Stab ist vorzugsweise voll ausgebildet. An mindestens einem seiner Enden kann der Stab abgerundet sein.

Das bioresorbierbare Material aus dem der Stab gefertigt wird, ist vorteilhafterweise im wesentlichen spröde und brüchig. Zweckmässigerweise weist das bioresorbierbare Material eine Bruchdehnung ε = (Δl x 100/L) < 10 % auf. Der Vorteil eines solchen Materials liegt in seiner besseren Resorbierbarkeit.

Gemäß Erfindung besteht der Stab aus einem verstärkten, vorzugsweise selbst-verstärkten bioresorbierbaren Material. Das bioresorbierbare Material kann ein Poly-L-Lactid (PLLA) oder ein Caprolacton sein. Diese Materialien weisen den Vorteil auf, dass sie schneller via Gelenkflüssigkeit resorbieren. Vorteilhafterweise besteht der Stab aus einem Copolymer der Milchsäure und Glykolsäure, vorzugsweise im Verhältnis von 3 : 1 bis 5 : 1. Der Stab kann auch aus einem Copolymer aus Poly-L-Lactide (PLLA) und Poly (DL-lactid-Co-Glycolid Acid) PLGA bestehen, vorzugsweise im Verhältnis von 3: 1 bis 5 : 1 und typischerweise im Verhältnis von 4:1. Der Stab kann auch aus einem Copolymer aus Poly-L,D-Lactid bestehen.

Ein Stab aus Poly-L-Lactid ist aus US-A-4 898 186 bekannt.

Die Länge des Stabes beträgt zweckmässigerweise maximal 6 cm, vorzugsweise maximal 5 cm. Die Länge beträgt zweckmässigerweise mindestens 3,5 cm, vorzugsweise mindestens 4 cm.

In einer weiteren Ausführungsform ist der Stab zur temporären Schienung von Zehen, insbesondere zur Behandlung von Hammerzehen oder anderen Fehlstellungen von Zehen adaptiert.

In wiederum einer weiteren Ausführungsform ist der Stab zur Fixierung von Gelenksfragmenten, insbesondere solchen mit Knochen - und Knorpelanteilen adaptiert.

In einer anderen Ausführungsform weist der Stab ein zur Einführung in den Knochen bestimmtes vorderes Ende auf, welches vorzugsweise stumpf, vorzugsweise planar ausgebildet ist. Die stumpfe Ausführung des vorderen Endes eignet sich besonders bei einer Anwendung im Falle von Osteochondritis. Eine spitze Ausführung des vorderen Endes ist hingegen im Falle einer Zehenanwendung besonders geeignet.

In wiederum einer anderen Ausführungsform umfasst diese ein Kopfteil, welches koaxial an den Stab angrenzt, ein dem Stab axial entgegengesetztes hinteres Ende und einen sich gegen das hintere Ende erweiternden Querschnitt umfasst. Die Vorteile einer solchen Ausführungsform liegen darin, dass bei einer Anwendung der Vorrichtung im Falle von Osteochondritis folgende Nageleffekte erzielt werden können:
A) durch die Reibung des Stabes auf einer relevanten Länge wird eine ausreichende Stabilität erreicht, während
B) im Kopfteil, wo die Reibung wegen dessen kurzer Länge schwach ist, der durch den Kanteneffekt des Kopfteils erzielte Formschluss die axiale Stabilität ergibt.

In einer weiteren Ausführungsform vergrössert sich die zur Zentralachse orthogonale Querschnittsfläche des Kopfteils progressiv gegen das hintere Ende des Kopfteils.

In wiederum einer weiteren Ausführungsform weist der Kopfteil eine zur Zentralachse koaxiale kreiszylindrische Hüllfläche mit einem dem maximalen Aussendurchmesser des Stabes entsprechenden Durchmesser auf.

In einer anderen Ausführungsform beträgt das Verhältnis zwischen der Länge I des Kopfteils und der Länge L des Stabes zwischen 1/20 und 1/3.

Die Erfindung und Weiterbildungen der Erfindung werden im folgenden anhand von teilweise schematischen Darstellungen zweier Ausführungsbeispiele noch näher erläutert.

Es zeigen:
Fig. 1 eine Seitenansicht des erfindungsgemässen Stabes;
Fig. 2 ein Querschnitt durch den Stab nach Fig. 1 längs der Linie II - II;
Fig. 3 eine perspektivische Ansicht einer Modifikation des Stabes nach Fig. 1 ;
Fig. 4 einen Querschnitt durch den Stab nach Fig. 3 längs der Linie IV-IV;
Fig. 5 eine Aufsicht auf eine Zehe mit endomedullär eingeführtem bioresorbierbarem Stab;
Fig. 6 eine perspektivische Ansicht einer weiteren Ausführungsform des erfindungsgemässen Stabes;
Fig. 7 eine Seitenansicht orthogonal zu einer Seitenfläche der in Fig. 6 dargestellten Ausführungsform; und
Fig. 8 einen Schnitt entlang der Linie III - III in Fig. 7.

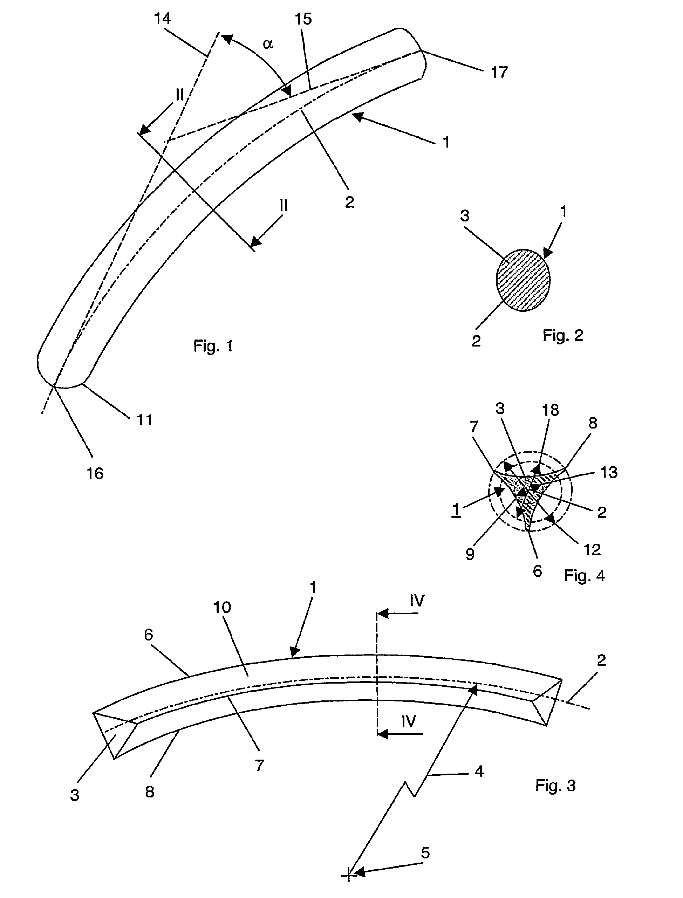

Die in den Fig. 1 und 2 dargestellte Vorrichtung zur temporären Schienung von Zehen, insbesondere zur Behandlung von Hammerzehen oder anderen Fehlstellungen von Zehen besteht im wesentlichen aus einem gekrümmten Stab 1 mit der Zentralachse 2 und einem unrunden (hier elliptischen) Querschnitt 3, weicher aus einem vorzugsweise selbstverstärkten Poly-L,D-Lactid (SR-PLA 96/4) besteht. Auch Copolymere aus Poly-L-Lactide (PLLA) und Poly-(DL-lactid-Co-Glycolid Acid) (PLGA), vorzugsweise im Verhältnis 4:1 eignen sich dazu. Auch ein Gemisch aus 96% Poly-L-Lactid (PLLA) mit 4% Poly-D-Lactid hat sich als vorteilhaft erwiesen.

Der Stab 1 weist eine in der Zeichenebene liegende Biegung mit einem Krümmungsradius der Länge 10 cm auf. Die Tangenten 14,15 an den beiden Endpunkten 16,17 der Zentralachse 2 des Stabes 1 schneiden sich dabei unter einem Winkel α von 10° bis 20°, typischerweise von 15°. Die Länge des Stabes 1 beträgt 3,75 cm. Die Oberfläche des Stabes 1 ist völlig glatt. Das eine Ende 16 des Stabes 1, welches zur Einführung in die Zehe bestimmt ist, ist verjüngt ausgebildet, so dass es in eine Abrundung 11 ausläuft.

In den Fig. 3 und 4 ist eine alternative Ausführungsform des Stabes 1 dargestellt, welche sich von der in Fig. 1 dargestellten Ausführungsform nur darin unterscheidet, dass der unrunde Querschnitt 3 statt elliptisch, dreieckförmig ausgebildet ist, so dass die Mantelfläche 10 des Stabes 1 drei Längskanten oder Längsgräte 6,7,8 aufweist. Die Seiten des Dreiecks sind dabei konkav ausgebildet, so dass die Längskanten oder Längsgräte 6,7,8 durch konkave Vertiefungen 9 voneinander getrennt sind. Der Stab 1 weist eine Krümmung auf mit einem Krümmungsradius 4 der Länge 10 - 15 cm, typischerweise von 12,5 cm. Der dreieckige Querschnitt 3 des Stabes 1 verjüngt sich dabei in Richtung des Zentrums 5 des Krümmungsradius 4.

Die im Markkanal der betroffenen Knochen vorzunehmende Aufbohrung weist einen Bohrungsdurchmesser 18 auf, der vorteilhafterweise kleiner ist als der maximale Aussendurchmesser 12 des Stabes 1, damit sich die Längsgräte 6,7,8 in die Wände des aufgebohrten Markkanals schneiden können, so dass eine Rotationsstabilität des Stabes 1 resultiert. Der Kerndurchmesser 13 des Stabes beträgt 1,0 - 2,5 mm, typischerweise 1,6 mm.

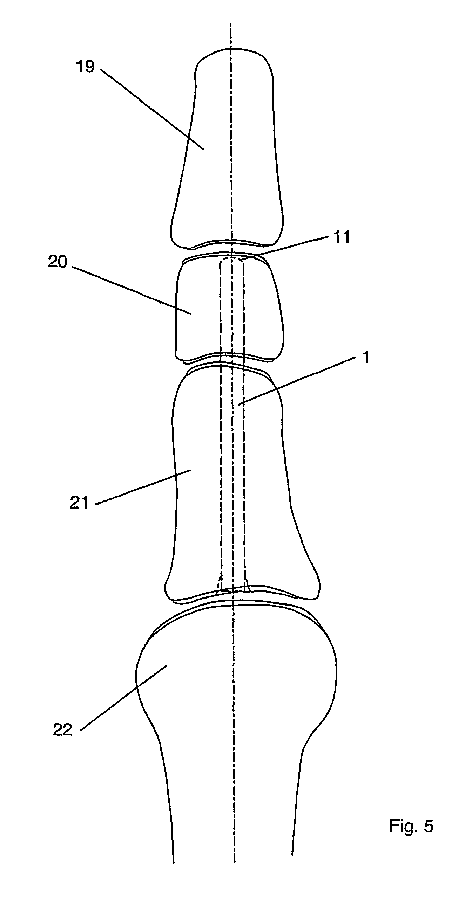

In Fig. 5 ist eine Zehe mit der Endphalanx 19, Mittelphalanx 20, Grundphalanx 21 und dem Metatarsaleköpfchen 22 dargestellt. Die Einführung des Stabes 1 erfolgt in die zuvor aufgebohrten Markkanäle der Mittelphalanx 20 und der Grundphalanx 21.

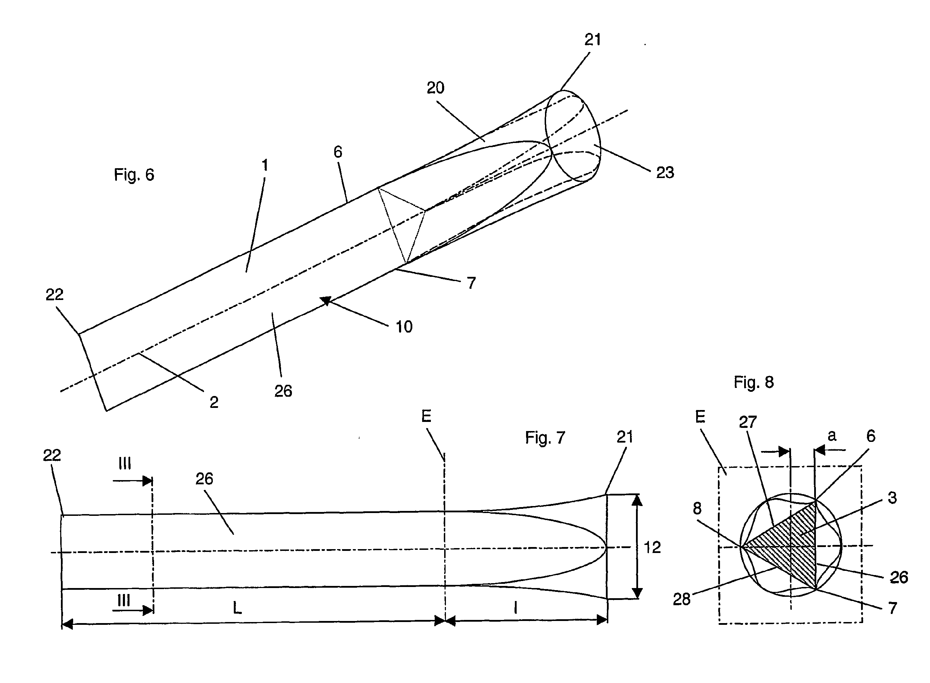

Die in den Fig. 6 bis 8 dargestellte Ausführungsform umfasst neben dem Stab 1 ein zur Zentralachse 2 koaxiales Kopfteil 20, wobei der Stab 1 und der Kopfteil 20 bezüglich einer zur Zentralachse 2 orthogonalen Ebene E asymmetrisch ausgebildet sind. Die Zentralachse 2, welche durch die Verbindungslinie der Schwerpunkte der axial aufeinanderfolgenden Querschnittsflächen gebildet wird, verläuft in der hier dargestellten Ausführungsform geradlinig. Der Stab 1 ist prismatisch ausgebildet, während der Kopfteil 20 einen sich gegen das hintere Ende 21 erweiternden Längsabschnitt bildet.

Der Stab 1, welcher durch das zur Einführung in die Zehe bestimmte vordere Ende 22 und die Ebene E begrenzt wird, weist auf seiner gesamten Länge L einen dreieckförmigen Querschnitt 3 auf, so dass die Mantelfläche 10 des Stabes drei ebene Seitenflächen 26, 27, 28 und drei Längskanten oder Längsgräte 6, 7, 8 aufweist. Der Querschnitt 3 wird durch ein gleichseitiges Dreieck begrenzt, welches einen Umkreis mit dem maximalen Aussendurchmesser 12 des Stabes 1 aufweist.

Der Kopfteil 20 zeichnet sich dadurch aus, dass die ebenen Seitenflächen 26, 27, 28 des Stabes 1 in axialer Richtung derart gekrümmt sind, dass der Abstand a zwischen der Zentralachse 2 und je einer der Seitenflächen 26, 27, 28 gegen das hintere Ende 21 des Kopfteils 20 progressiv zunimmt. Ferner weist der Kopfteil 20 eine Länge I und eine kreiszylindrische Hüllfläche mit dem maximalen Aussendurchmesser 12 auf, in welche die Seitenflächen 26, 27, 28 am hinteren Ende 21 des Stabes 1 münden, so dass die zur Zentralachse 2 orthogonale Stirnfläche 23 am hinteren Ende 21 des Stabes 1 eine Kreisfläche ist.

Zum besseren Verständnis der erfindungsgemässen Vorrichtung folgt ein stichwortartiger Operationsablauf:
1. Der Patient wird in Rückenlage gebracht.
2. Es erfolgt ein dorsaler Zugang mit Längsschnitt von der Mittelphalanx 20 bis zum Metatarsaleköpfchen 22.
3. Exzision oder einfache Längsspaltung der langen Strecksehne.
4. Eröffnung des proximalen Interphalangeal-Gelenks.
5. Exzision des Interphalangeal-Gelenks.
6. Beugung der distalen Zehe.
7. Aufbohren des Markkanals der Mittelphalanx 20 bis zur distalen Epiphyse.
8. Eröffnung des Metatarso-Phalangeal-Gelenkes auf drei Viertel des Umfangs.
9. Es verbleibt die plantare Gelenksplatte.
10. Beugung der Grundphalanx 21.
11.Aufbohren des Markkanals durch die gesamte Grundphalanx 21.
12. Einschlagen des Stabes 1 von proximal nach distal durch das vorgebohrte Loch mit dem Bohrungsdurchmesser 18 im Markkanal bis zum distalen Ende der Mittelphalanx 20.
13.Abschneiden des Stabes 1 auf Höhe der Gelenksfläche der Grundphalanx 21.
14.Anbringen der Hautnaht.

### Beschreibung für eine Gelenksfragment-Fixierung

Am Beispiel einer Osteochondritis dissecans tali:
1. Osteotomie des medialen Malleolus
2. Prüfen der Instabilität des osteochondralen Fragmentes oder Reposition
3. Anbohren des Fragmentes und des Taluskörpers und Tiefenmessung
4. Einschlagen des Stabes
5. Absägen des Stabes auf Knorpelhöhe.

Das Profil des Fragmentes erbringt die notwendige Rotationsstabilität. Das Einbringen eines zweiten Stabes erübrigt sich. Ohnehin ist meist auch kein Platz dafür da und ein solcher zweiter Stab würde zusätzlich die Vitalität (Durchblutung) gefährden.

## Patentansprüche

1. Vorrichtung für die Osteosynthese, welche einen Stab (1) mit der Zentralachse (2) und der Länge L umfasst, der zum überwiegenden Teil aus einem bioresorbierbaren Material besteht und der mindestens in einem Bereich der Länge L einen zur Zentralachse (2) orthogonalen, unrunden Querschnitt (3) aufweist,
**dadurch gekennzeichnet, dass**
A) der Stab (1) aus einem der folgenden Materialien besteht:
- einem Copolymer der Milchsäure und Glykolsäure;
- einem Copolymer aus Poly-L-Lactide [PLLA]) und Poly (DL-lactid-Co-Glycolid Acid) [PLGA];
- einem Copolymer aus Poly-L,D-Lactid; oder
- einem Caprolacton; und
B) der Stab (1) auf seiner Mantelfläche mindestens drei Längskanten oder Längsgräte (6,7,8) aufweist, welche durch konkave Vertiefungen (9) voneinander getrennt sind.

2. Vorrichtung nach Anspruch 1, **dadurch gekennzeichnet, dass** der Stab (1) voll ausgebildet ist.

3. Vorrichtung nach Anspruch 1 oder 2, **dadurch gekennzeichnet, dass** der unrunde Querschnitt (3) polygonal, vorzugsweise dreieckig ausgebildet ist.

4. Vorrichtung nach einem der Ansprüche 1 bis 3, **dadurch gekennzeichnet, dass** die Zentralachse (2) des Stabes (1) gekrümmt ist, vorzugsweise mit einem Krümmungsradius (4) im Bereich von 10 - 15 cm.

5. Vorrichtung nach Anspruch 4, **dadurch gekennzeichnet, dass** sich die Tangenten (14,15) an den beiden Endpunkten (16,17) der Zentralachse (2) des Stabes (1) unter einem Winkel von 5° - 20° schneiden.

6. Vorrichtung nach einem der Ansprüche 1 bis 5, **dadurch gekennzeichnet, dass** der maximale Aussendurchmesser (12) des Stabes (1) 1,5 - 3,5 mm beträgt.

7. Vorrichtung nach einem der Ansprüche 1 bis 6, **dadurch gekennzeichnet, dass** der Kerndurchmesser (13) des Stabes (1) 1,0 - 2,5 mm beträgt.

8. Vorrichtung nach einem der Ansprüche 4 - 7, **dadurch gekennzeichnet, dass** sich der Stab (1) in Richtung des Zentrums (5) des Krümmungsradius (4) der Zentralachse (2) verjüngt.

9. Vorrichtung nach einem der Ansprüche 1 bis 8, **dadurch gekennzeichnet, dass** er zur temporären Schienung von Zehen, insbesondere zur Behandlung von Hammerzehen oder anderen Fehlstellungen von Zehen adaptiert ist.

10. Vorrichtung nach einem der Ansprüche 1 bis 8, **dadurch gekennzeichnet, dass** er zur Fixierung von Gelenksfragmenten, insbesondere solchen mit Knochen - und Knorpelanteilen adaptiert ist.

11. Vorrichtung nach einem der Ansprüche 1 bis 10, **dadurch gekennzeichnet, dass** der Stab (1) ein zur Einführung in den Knochen bestimmtes vorderes Ende (22) aufweist, welches vorzugsweise stumpf, vorzugsweise planar ausgebildet ist.

12. Vorrichtung nach einem der Ansprüche 1 bis 11, **dadurch gekennzeichnet, dass** koaxial an den Stab (1) ein Kopfteil (20) angrenzt, welcher ein dem Stab (1) axial entgegengesetztes hinteres Ende (21) hat und einen sich gegen das hintere Ende (21) erweiternden Querschnitt (3) aufweist.

13. Vorrichtung nach Anspruch 12, **dadurch gekennzeichnet, dass** sich die zur Zentralachse (2) orthogonale Querschnittsfläche des Kopfteils (20) gegen das hintere Ende (21) progressiv vergrössert.

14. Vorrichtung nach Anspruch 12 oder 13, **dadurch gekennzeichnet, dass** der Kopfteil (20) eine zur Zentralachse (2) koaxiale kreiszylindrische Hüllfläche mit einem dem maximalen Aussendurchmesser (12) des Stabes (1) entsprechenden Durchmesser aufweist.

15. Vorrichtung nach einem der Ansprüche 12 bis 14, **dadurch gekennzeichnet, dass** das Verhältnis zwischen der Länge I des Kopfteils (20) und der Länge L des Stabes (1) zwischen 1/20 und 1/3 beträgt.

16. Kit mit einer Vorrichtung nach einem der Ansprüche 1 bis 15 und mit einem Bohrer, **dadurch gekennzeichnet, dass** der Bohrer einen Durchmesser D aufweist, welcher im Vergleich zum maximalen Aussendurchmesser (12) des Stabes (1) kleiner ist und vorzugsweise mehr als 30 % kleiner ist.

17. Vorrichtung nach einem der Ansprüche 1 bis 15 geeignet zur Behandlung von:
- Gelenksfragmenten, insbesondere bei Osteochondrosis oder Osteochondritis; und
- zur temporären Schienung von Zehen, insbesondere von Hammerzehen.

## Claims

1. Osteosynthesis device comprising a pin (1) with a central axis (2) and a length L, predominantly consisting of a bioresorbable material, the pin (1) having, in at least a section of its length L, a non-circular cross section (3) orthogonal to the central axis (2),
**characterized in that**
A) the pin (1) consists of one of the following materials:
- a copolymer of lactic acid and glycol acid;
- a copolymer of poly-L-lactide (PLLA) and poly (DL-lactide-co-glycolic acid) (PLGA);
- a copolymer of poly-L,D-lactide; or
- a caprolactone; and
B) the pin (1) presents on its peripheral surface at least three longitudinal edges or longitudinal ridges (6, 7, 8) which are separated from each other by concave depressions (9).

2. Device according to claim 1, **characterized in that** the pin (1) is solid.

3. Device according to claim 1 or 2, **characterized in that** the non-circular cross section (3) is formed in a polygonal and preferably triangular shape.

4. Device according to claim 1 to 3, **characterized in that** the central axis (2) of the pin (1) is curved, preferably with a radius of curvature (4) in the range of 10 - 15 cm.

5. Device according to claim 4, **characterized in that** the tangents (14, 15) at the two endpoints (16, 17) of the central axis (2) of the pin (1) intersect under an angle of 5° - 20°.

6. Device according to one of the claims from 1 to 5, **characterized in that** the maximum outside diameter (12) of the pin (1) amounts to 1.5 - 3.5 mm.

7. Device according to one of the claims from 1 to 6, **characterized in that** the core diameter (13) of the pin (1) amounts to 1.0 - 2.5 mm.

8. Device according to one of the claims from 4 to 7, **characterized in that** the pin (1) tapers in the direction of the centre (5) of the radius of curvature (4) of the central axis (2).

9. Device according to one of the claims from 1 to 8, **characterized in that** it is adapted for the temporary splinting of toes, in particular for the treatment of hammer toes or other toe misalignments.

10. Device according to one of the claims from 1 to 8, **characterized in that** it is adapted for the fixation of joint fragments, in particular those with bone and cartilage portions.

11. Device according to one of the claims from 1 to 10, **characterized in that** the pin (1) presents a front end (22) destined for inserting into the bone, which is formed in a blunt and preferably planar manner.

12. Device according to one of the claims from 1 to 11, **characterized in that** the pin (1) is coaxially adjoined by a head portion (20) having a rear end (21) axially opposed to the pin (1) and a cross section (3) that enlarges in a direction toward the rear end (21).

13. Device according to claim 12, **characterized in that** the cross sectional surface of the head portion (20) orthogonal to the central axis (2) gradually increases in a direction toward the rear end (21).

14. Device according to claim 12 or 13, **characterized in that** the head portion (20) presents a circularly cylindrical enveloping surface coaxial with the central axis (2) with a diameter corresponding to the maximum outside diameter (12) of the pin (1).

15. Device according to one of the claims from 12 to 14, **characterized in that** the ratio between the length I of the head portion (20) and the length L of the pin (1) lies between 1/20 and 1/3.

16. A kit with a device according to one of the claims from 1 to 15 and a drill, **characterized in that** the drill has a diameter D which is smaller, preferably over 30% smaller than the maximum outside diameter (12) of the pin (1).

17. Device according to one of the claims from 1 to 15 suitable for the treatment of:
- joint fragments, in particular in the case of osteochondrosis or osteochondritis; and
- for temporary splinting of toes, in particular of hammer toes.

## Revendications

1. Dispositif d'ostéosynthèse qui comprend une tige (1), avec l'axe central (2) et de longueur (L), qui est en majeure partie composée d'un matériau biorésorbable et qui, au moins dans une zone de longueur (L), présente une section transversale (3) non circulaire orthogonale à l'axe central (2), **caractérisé en ce que**
A) la tige (1) est composée d'un des matériaux suivants :
- un copolymère d'acide lactique et d'acide glycolique ;
- un copolymère de poly-L-lactide (PLLA) et de poly (acide DL-lactide-co-glycolide)[PLGA] ;
- un copolymère de poly-L,D-lactide ; ou
- une caprolactone ; et
B) la tige (1) présente sur sa surface d'enveloppe au moins trois arêtes longitudinales ou nervures longitudinales (6, 7, 8) qui sont séparées entre elles par des creux (9) concaves.

2. Dispositif selon la revendication 1, **caractérisé en ce que** la tige (1) est constituée de façon pleine.

3. Dispositif selon la revendication 1 ou 2, **caractérisé en ce que** la section transversale (3) non circulaire est constituée de façon polygonale, de préférence de façon triangulaire.

4. Dispositif selon une des revendications 1 à 3, **caractérisé en ce que** l'axe central (2) de la tige (1) est courbe, de préférence avec un rayon de courbure (4) dans la plage de 10 - 15 cm.

5. Dispositif selon la revendication 4, **caractérisé en ce que** les tangentes (14, 15) se coupent aux deux points extrêmes (16, 17) de l'axe central (2) de la tige (1) à un angle de 5 ° - 20 °.

6. Dispositif selon une des revendications 1 à 5, **caractérisé en ce que** le diamètre extérieur (12) maximal de la tige (1) est de 1,5 - 3,5 mm.

7. Dispositif selon une des revendications 1 à 6, **caractérisé en ce que** le diamètre de noyau (13) de la tige (1) est de 1,0 - 2,5 mm.

8. Dispositif selon une des revendications 4 à 7, **caractérisé en ce que** la tige (1) se rétrécit en direction du centre (5) du rayon de courbure (4) de l'axe central (2).

9. Dispositif selon une des revendications 1 à 8, **caractérisé en ce qu'**il est adapté pour la stabilisation temporaire d'orteils, en particulier pour le traitement d'orteils en marteau ou d'autres mauvais positionnements d'orteils.

10. Dispositif selon une des revendications 1 à 8, **caractérisé en ce qu'**il est adapté pour la fixation de fragments d'articulation, en particulier de ceux comprenant des fractions osseuses ou cartilagineuses.

11. Dispositif selon une des revendications 1 à 10, **caractérisé en ce que** la tige (1) présente une extrémité avant (22) qui est destinée à être introduite dans l'os et qui est constituée de préférence de façon non pointue et de préférence de façon plane.

12. Dispositif selon une des revendications 1 à 11, **caractérisé en ce qu'**une partie de tête (20) est adjacente de façon coaxiale à la tige (1) et a une extrémité arrière (21) opposée dans le sens axial à la tige (1) et présente une section transversale (3) qui s'élargit vers l'extrémité arrière (21).

13. Dispositif selon la revendication 12, **caractérisé en ce que** la surface de section transversale, orthogonale à l'axe central (2), de la partie de tête (20) augmente progressivement vers l'extrémité arrière (21).

14. Dispositif selon la revendication 12 ou 13, **caractérisé en ce que** la partie de tête (20) présente une surface enveloppante cylindrique circulaire coaxiale à l'axe central (2) avec un diamètre correspondant au diamètre extérieur (12) maximal de la tige (1).

15. Dispositif selon une des revendications 12 à 14, **caractérisé en ce que** le rapport entre la longueur I de la partie de tête (20) et la longueur L de la tige (1) est compris entre 1/20 et 1/3.

16. Kit avec un dispositif selon une des revendications 1 à 15 et avec un foret, **caractérisé en ce que** le foret présente un diamètre D qui, comparé au diamètre extérieur (12) maximal de la tige (1), est inférieur, et est préférence inférieur de plus de 30 %.

17. Dispositif selon une des revendications 1 à 15, adapté pour le traitement de:
- fragments d'articulation, en particulier en cas d'ostéochondrose ou d'ostéochondrite ; et
- à la stabilisation temporaire d'orteils, en particulier d'orteils en marteau.
